# EUROPEAN PATENT APPLICATION

(11) **EP 1 314 428 A1**
(43) Date of publication of application: **28.05.2003**
(21) Application number: 01961146.6
(22) Date of filing: 29.08.2001
(51) Int. Cl.: A61K 31/593, A61K 47/02, A61K 9/08

(54) **OCT PREPARATIONS**

(30) Priority: 30.08.2000 JP 2000260149
(71) Applicant: CHUGAI SEIYAKU KABUSHIKI KAISHA, Tokyo, 115-8543 (JP)
(72) Inventor: FUJIMOTO, Ichiro, c/o Chugai Seiyaku K.K., Tokyo 115-8543 (JP)
(74) Representative: HOFFMANN - EITLE
(86) International application number: JP0107399
(87) International publication number: WO02017932

(57) **Abstract**

An object of the present invention is to provide OCT formulations showing excellent stability.

According to the present invention, OCT formulations comprising 1α,25-dihydroxy-22-oxavitamin D₃ (OCT) and a pH modifier are provided.

## Description

### FIELD OF THE INVENTION

The present invention relates to formulations containing 1α.25-dihydroxy-22-oxavitamin D₃ (1α, 3β-dihydroxy-20α- (3-hydroxy-3-methylbutyloxy) -9, 10-seco-5, 7,10 (19)-pregnatriene, hereinafter also referred to as OCT) as an active ingredient.

### BACKGROUND ART

Vitamin D derivatives have various physiological effects and are expected as therapeutic agents for various diseases. Especially, the 22-oxa analogue of 1α,25-dihydroxyvitamin D₃, 1α,25-dihydroxy-22-oxavitamin D₃ has a strong in vitro differentiation-inducing activity and a low in vivo calcium elevating effect and has been clinically tested for its effects as a candidate for treating secondary hyperparathyroidism, psoriasis and other diseases.

Generally, vitamin D derivatives are chemically unstable. OCT mentioned above is known to rapidly decompose especially in aqueous solutions, and various formulation designs have been made to supply stable OCT formulations to the market. For example, known formulations of vitamin D derivatives include injectable compositions disclosed in JPA No. 149619/87 and CALCIJEX (trade name of Abbott, USA), but all of them contained chelating agents such as EDTA or antioxidants such as sodium ascorbate to stabilize vitamin D derivatives.

These formulations containing stabilizing components such as chelating agents or antioxidants may have the possibility of interaction between the stabilizing components and biological components such as various ions. Therefore, Drug Manufacturing Guidelines ("The 2000 Drug Manufacturing Guidelines" published April 25, 2000 by Jiho, Inc. under the supervision of Pharmaceutical Examination Institute) limited inclusion of stabilizers in injections for prolonged chronic use.

### DISCLOSURE OF THE INVENTION

As described above, existing formulations of vitamin D derivatives could not contain sufficiently stabilized vitamin D derivatives. An object of the present invention is to provide OCT formulations containing stabilized active ingredients with a decreased loss of active ingredients even during long-term storage. Another object of the present invention is to provide OCT formulations in which active ingredients can be stabilized with substantially no stabilizing components such as chelating agents and antioxidants.

As-a-result of careful studies, the inventor accomplished the present invention on the basis of the finding that OCT is stabilized on the alkaline side in aqueous solutions.

According to the present invention, OCT formulations comprising 1α. 25-dihydroxy-22-oxavitamin D₃ (OCT) and a pH modifier are provided.

The pH modifier preferably comprises disodium hydrogenphosphate and sodium dihydrogenphosphate.

Preferably, OCT formulations of the present invention have a pH of 7.5-8.5.

Preferably, OCT formulations are substantially free from chelating agents and/or antioxidants as stabilizers.

In addition to the components mentioned above, formulations of the present invention may contain solubilizing agents, adsorption inhibitors, isotonizing agents or the like.

The present application claims priority based on Japanese Patent Application No. 260149/2000, the disclosure of which is wholly incorporated herein as a reference.

### PREFERRED MODE FOR CARRYING OUT THE INVENTION

The active ingredient of formulations of the present invention OCT is a known product that can be prepared by known processes. For example, it can be prepared by the processes described in JPA No. 072994/94 (published on March 15, 1994), JPA No. 080626/94 (published on March 22, 1994), JPA No. 256300/94 (published on September 13, 1994), Kubodera et al. (Bioorganic & Medicinal Chemistry Letters, 4(5): 753-765, 1994) and WO98/09935, but desirably should be highly purified.

The OCT concentration in OCT formulations of the present invention is 0.1 µg/ml -100 µg/ml, preferably 0.5 µg/ml - 50 µg/ml, more preferably 1.0 µg/ml - 20 µg/ml.

Preferably, OCT formulations of the present invention are free from chelating agents such as EDTA. Desirably, they are also free from antioxidants such as ascorbates.

Solvents for OCT formulations of the present invention include water for injection, sterile physiological saline solution for injection, Ringer's solution for injection, glucose solution, etc., preferably water for injection. The proportion of solvents to the total OCT formulations is preferably 50% (v/v) - 99.99% (v/v), more preferably 90% (v/v) - 99.9% (v/v).

Generally, vitamin D derivatives are less soluble in water, but readily soluble in organic solvents. Therefore, solubilizing agents are preferably included when water is used as a solvent for OCT formulations of the present invention. Solubilizing agents include alcohols such as anhydrous ethanol as well as sodium salicylate, polyethylene glycol and propylene glycol, but preferably anhydrous ethanol in terms of biological influence, availability and other reasons. They are contained in an amount of 0.001% (v/v) - 20% (v/v), preferably 0.005% (v/v) - 2% (v/v) of the total formulations.

As described above, the inventor found that OCT is stable in aqueous alkaline solutions. Thus, OCT formulations of the present invention contain a pH modifier to stabilize OCT in solutions and to adjust the pH of the solutions in a physiologically acceptable range. Such pH modifiers include inorganic acids such as hydrochloric acid and phosphoric acid; organic acids such as acetic acid, succinic acid, tartaric acid and acidic amino acids; inorganic alkalis such as sodium hydroxide; organic alkalis such as basic amino acids; as well as sodium dihydrogenphosphate, disodium hydrogenphosphate, dipotassium hydrogenphosphate, potassium dihydrogenphosphate and glycine. In the present invention, a combination of sodium dihydrogenphosphate and disodium hydrogenphosphate is especially preferred.

As described later, stability of OCT in OCT formulations of the present invention after storage at 25°C, 40°C and 50°C for 4 weeks is high at pH 7.0 or more, especially 7.5 or more. Considering stimulation to humans, the pH of OCT formulations is preferably closer to neutral, specifically pH 9.0 or less, and especially 8.5 or less. In brief, the pH of OCT formulations is preferably about 7.0-9.0, more preferably about 7.5-8.5, most preferably about 8.0.

OCT formulations of the present invention preferably contain an adsorption inhibitor because OCT tends to be adsorbed to the glass wall when it is filled as an aqueous solution in a glass container such as an ampule, vial or syringe or it tends to be adsorbed to the filter when it is aseptically filtered during formulation processes. Such adsorption inhibitors include surfactants, preferably nonionic surfactants such as polysorbate 20, polysorbate 80, and polyoxyethylene derivatives. In terms of biomedical safety, availability and other reasons, polysorbate 20 is especially preferred. Adsorption inhibitors are contained in an amount of 0.001% by weight - 0.5% by weight, preferably 0.005% by weight - 0.1% by weight of the total OCT formulations.

OCT formulations of the present invention preferably contain an isotonizing agent to reduce biological influence by controlling osmosis. For example, sodium chloride is preferably used. It is contained in an amount of 0.1% by weight - 2% by weight, preferably 0.2% by weight - 1% by weight of the total formulations.

OCT formulations of the present invention may contain soothing agents such as benzyl alcohol, lidocaine and procaine; preservatives such as paraoxybenzoic acid, chlorobutanol, benzalkonium chloride, thimerosal, etc.

OCT formulations of the present invention are preferably in the form of a solution formulation, but may also be in other dosage forms such as freeze-dried formulations or spray-dried formulations.

OCT formulations of the present invention can be prepared by any known aseptic process, for example, as follows.

In a solubilizing agent is dissolved OCT optionally followed by an adsorption inhibitor. The resulting solution is adjusted to a desired pH with a pH modifier. Here, the pH modifier preferably has been preliminarily dissolved in a solvent before use. A solvent is added to the pH-adjusted solution up to a final volume and the mixed solution is stirred. The resulting OCT solution is aseptically filtered and packed in a preliminarily sterilized storage container. These steps preferably-take place at room temperature. In order to avoid the effects of light during storage, an amber storage container is preferably used.

OCT formulations of the present invention are preferably packed in a sealed and sterilized plastic or glass container. The container may be in the form having a defined volume such as ampules, vials or disposable syringes or a large volume such as injection bags or bottles.

OCT formulations of the present invention are normally administered via parenteral routes such as injection (intracutaneous injection, subcutaneous injection, intravenous injection, muscle injection, etc.) or percutaneous, mucosal, nasal or pulmonary route, but may also be orally administered.

The dose of OCT formulations of the present invention can be determined depending on the type of disease to be treated, the severity of the disease, the age, body weight and sex of the patient and other factor. For example, a formulation containing 0.1-1000 µg. preferably 1-500 µg of OCT is administered once to seven times weekly.

### EXAMPLES

The following. Examples further illustrate the present invention without, however, limiting the invention thereto.

### Example 1

To 950 mL of water for injection were added 19.2 g of disodium hydrogenphosphate/12H₂O (Na₂HPO₄/12H₂O), 0.44 g of sodium dihydrogenphosphate/2H₂O (NaH₂PO₄/2H₂O) and 5.0 g of sodium chloride, and the mixture was stirred to dissolution to give a phosphate buffer, pH 8.0.

To 2 mL of anhydrous ethanol was added 2.0 mg of OCT, and the mixture was stirred to dissolution and then further stirred with 1 mL of an aqueous 10% w/v polysorbate 20 solution. Then, the mixed solution was stirred with 950 mL of the above phosphate buffer, and then water for injection was added up to 1000 mL and the mixed solution was further stirred. The resulting OCT formulated solution had a phosphate concentration of about 56 mM.

The OCT formulated solution was aseptically filtered through a barrier filter and then 1 mL of the filtrate was packed in each sterilized amber glass vial, which was stopped with a sterilized rubber plug. Then, an aluminum cap was screwed onto the vial. All these steps were aseptically performed at room temperature.

### Example 2

OCT formulations were prepared in the same manner as in Example 1 except that phosphate buffers were prepared according to the formulae below at pH values of 6.5, 7.0, 7.5, 8.5 and 9.0.

| pH 6.5 | |
|---|---|
| Disodium hydrogenphosphate/12H₂0 | 7.64 g |
| Sodium dihydrogenphosphate/2H₂O | 5.49g |

| pH 7.0 | |
|---|---|
| Disodium hydrogenphosphate/12H₂O | 12.6 g |
| Sodium dihydrogenphosphate/2H₂O | 3.30 g |

| pH 7.5 | |
|---|---|
| Disodium hydrogenphosphate/12H₂O | 16.7 g |
| Sodium dihydrogenphosphate/2H₂O | 1.56 g |

| pH 8.5 | |
|---|---|
| Disodium hydrogenphosphate/12H₂O | 19.2 g |
| Sodium dihydrogenphosphate/2H₂O | 0.13 g |

| pH 9.0 | |
|---|---|
| Disodium hydrogenphosphate/12H₂O | 19.2 g |
| Sodium dihydrogenphosphate/2H₂O | 0.02 g. |

These OCT formulation vials at various pH values and the OCT formulation vial at pH 8.0 obtained in Example 1 were allowed to stand in an incubator at 25°C, 40°C and 50°C for 4 weeks.

OCT formulations in various vials after storage for 4 weeks were taken as samples, which were measured for OCT content by reverse-phase high-speed liquid chromatography in a UV detector (wavelength 265 run). The percentage (%) of the OCT content remaining in each vial after storage for 4 weeks was calculated on the basis of the OCT content in the vial immediately after preparation.The results are shown in Table 1.

**Table 1**

| | Initial (%) | 25°C, 4 weeks (%) | 40°C, 4 weeks (%) | 50°C, 4 weeks (%) |
|---|---|---|---|---|
| pH 6.5 | 100.0 | 95.0 | 63.8 | 30.4 |
| pH 7.0 | 100.0 | 96.8 | 84.6 | 66.5 |
| pH 7.5 | 100.0 | 99.4 | 95.7 | 89.1 |
| pH 8.0 | 100.0 | 101.3 | 99.6 | 97.1 |
| pH 8.5 | 100.0 | 98.9 | 95.8 | 94.1 |
| pH 9.0 | 100.0 | 99.1 | 95.5 | 93.5 |

As apparent from Table 1, vials at pH 6.5 and pH 7.0 had a tendency to show a decreased OCT content even after storage at 25°C for 4 weeks and this tendency increased as storage temperature rose. Vials at pH 7.5 showed an OCT content loss of about 10% after storage at 50°C for 4 weeks. Vials at pH 8.5 and pH 9.0 had a tendency to show a slightly decreased OCT content as compared with vial at pH 8.0 after storage at 50°C for 4 weeks.

### Example 3

OCT formulations were prepared in the same manner as in Example 1 except that the amounts of OCT were adjusted to OCT concentrations of 1 µg/mL and 20 µg/mL and that the resulting formulations were-packed-in-sterilized amber glass ampules (1 mL/ampule).

Each of the resulting OCT formulation ampules was allowed to stand in an incubator at 40°C for one month and then measured for OCT content to calculate the percentage of the remaining OCT content (%). The results are shown in Table 2.

**Table 2**

| OCT concentration | 1 µg/mL | 20 µg/mL |
|---|---|---|
| Initial content | 100.0% | 100.0% |
| Content after 1 month at 40°C | 96.1% | 98.3% |

Table 2 shows no difference in the percentage of the remaining OCT content between OCT formulations at concentrations of 1 µg/mL and 20 µg/mL.

### INDUSTRY, APPLICABILITY

As has been described above, OCT formulations of the present invention have excellent storage stability because OCT is stabilized in aqueous solutions.

## Claims

1. An OCT formulation-comprising 1α,25-dihydroxy-22-oxavitamin D₃ (OCT) and a pH modifier.

2. The OCT formulation of Claim 1 wherein the pH modifier comprises disodium hydrogenphosphate and sodium dihydrogenphosphate.

3. The OCT formulation of Claim 1 or 2, which has a pH of 7.5-8.5.

4. The OCT formulation of any one of Claims 1 to 3, which is substantially free from chelating agents and/or antioxidants as stabilizers.
